# EUROPEAN PATENT APPLICATION

(11) **EP 4 751 735 A2**
(43) Date of publication of application: **03.06.2026**
(21) Application number: 26158282.9
(22) Date of filing: 05.01.2021
(51) Int. Cl.: A61K 45/06

(54) **TREATMENT OF MEMBRANOUS NEPHROPATHY, IGG4-RELATED DISEASE, AND ANTIPHOSPHOLIPID SYNDROME USING BTK INHIBITOR 2-[(3R)-3-[4-AMINO-3-(2-FLUORO-4-PHENOXY-PHENYL)PYRAZOLO[3,4-D]PYRIMIDIN-1-YL]PIPERIDINE-1-CARBONYL]-4-METHYL-4-[4-(OXETAN-3-YL)PIPERAZIN-1-YL]PENT-2-ENENITRILE**

(30) Priority: 06.01.2020 US 202062957724 P; 15.06.2020 US 202063039200 P
(62) Divisional of application: 21702343.1
(71) Applicant: Principia Biopharma Inc., Morristown, NJ 07960 (US)
(72) Inventor: NUNN, Philip, Massachusetts, 02141 (US); LANGRISH, Claire, Massachusetts, 02141 (US); THOMAS, Dolca, Massachusetts, 02141 (US)
(74) Representative: J A Kemp LLP

(57) **Abstract**

The present disclosure provides methods of treating a disease chosen from membranous nephropathy (MN), IgG4-related diseases, and antiphospholipid syndrome (APS) in a mammal using, *e.g.,* a therapeutically effective amount of, the BTK inhibitor 2-[(3R)-3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile or a pharmaceutically acceptable salt thereof.

## Description

This application claims the benefit of priority to U.S. Provisional Application No. 62/957,724, filed January 6, 2020, and U.S. Provisional Application No. 63/039,200, filed June 15, 2020, the contents of each of which are incorporated by reference herein in their entirety.

The present disclosure provides methods of treating a disease chosen from membranous nephropathy (MN), IgG4-related diseases, and antiphospholipid syndrome (APS) using a therapeutically effective amount of 2-[(3R)-3-|4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl|pent-2-enenitrile (also referred to herein as Compound (I), PRN1008. and rilzabrutinib). having the structure: 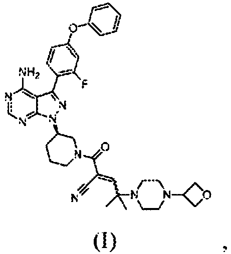 or a pharmaceutically acceptable salt thereof. The 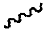 line at the alkene carbon in Compound (1) denotes that Compound (1) or a pharmaceutically acceptable salt thereof can be a (E) isomer, a (Z) isomer, or a mixture of (E) and (Z) isomers.

Compound (I) is disclosed in Example 31 of the PCT Application No. PCT/US2013/058614, filed on September 6, 2013. The disclosed synthesis provides Compound (1) requiring purification by column chromatography and affording a foam upon removal of solvent which can be crushed to obtain a powder.

Compound (1) is also known as PRN 1008 and rilzabrutinib. Compound (I) and pharmaceutically acceptable salts thereof are potent Bruton Tyrosine Kinase (BTK) inhibitors. The enzyme BTK is a member of the Tec family non-receptor tyrosine kinases. BTK is expressed in most hematopoietic cells, including B cells, mast cells, and macrophages. BTK plays a role in the development and activation of B cells. BTK activity has been implicated in the pathogenesis of several disorders and conditions, such as B cell-related hematological cancers (*e.g*., non-Hodgkin lymphoma and B cell chronic lymphocytic leukemia) and immune-mediated diseases (*e.g*., rheumatoid arthritis, graft versus host disease, Sjogren's syndrome, pemphigus, IBD, lupus, and asthma).

In some embodiments of the present disclosure, at least about 80% w/w, at least about 85% w/w, at least about 90% w/w, at least about 95% w/w, at least about 96% w/w, at least about 97% w/w, or at least about 99% w/w of Compound (I) or a pharmaceutically acceptable salt thereof is the (E) isomer. The ratio of the (E) to (Z) isomer can be calculated by methods well known in the art. One such method is HPLC total area normalization method.

In some embodiments, the mammal treated using Compound (1) or a pharmaceutically acceptable salt thereof is treatment naive with respect to a immunosuppressive therapy. In some embodiments, the mammal treated using Compound (I) or a pharmaceutically acceptable salt thereof is treatment naive with respect to any immunosuppressive therapy. In some embodiments, the mammal is a human.

In some embodiments, the mammal has been previously treated with an immunosuppressive therapy. In some embodiments, the mammal is a human.

In some embodiments, the present disclosure provides methods of using Compound (I) or a pharmaceutically acceptable salt thereof as a replacement therapy for an immunosuppressive therapy for a disease chosen from membranous nephropathy (MN), IgG4-related diseases, and antiphospholipid syndrome (APS). In some embodiments. the present disclosure provides methods of using Compound (I) or a pharmaceutically acceptable salt thereof as a replacement therapy for a disease chosen from membranous nephropathy (MN), IgG4-related diseases, and antiphospholipid syndrome (APS), wherein an immunosuppressant is used as a first or second line therapy for the disease. In some embodiments, Compound (I) or a pharmaceutically acceptable salt thereof is used in place of the immunosuppressive therapy. In some embodiments, Compound (I) or a pharmaceutically acceptable salt thereof is used in combination with the immunosuppressive therapy. In some embodiments, the present disclosure provides methods of using Compound (I) or a pharmaceutically acceptable salt thereof as a replacement therapy for a disease chosen from membranous nephropathy (MN), IgG4-related diseases, and antiphospholipid syndrome (APS), wherein an immunosuppressant is used as a first or second line maintenance therapy for the disease. In some embodiments, Compound (1) or a pharmaceutically acceptable salt thereof is used in place of the immunosuppressant. In some embodiments, Compound (I) or a pharmaceutically acceptable salt thereof is used in combination with the immunosuppressant.

In some embodiments, the present disclosure provides methods of eliminating or reducing a therapeutic dose of an immunosuppressive therapy used in chronic maintenance therapy in the treatment of a disease chosen from membranous nephropathy (MN). IgG4-related diseases, and antiphospholipid syndrome (APS) in a mammal in need thereof, wherein the immunosuppressive therapy is used as a first or second line treatment, comprising administering to said mammal in need of said treatment a therapeutically effective amount of Compound (I) or a pharmaceutically acceptable salt thereof. In some embodiments, Compound (I) or a pharmaceutically acceptable salt thereof is used in place of the immunosuppressive therapy. In some embodiments, Compound (1) or a pharmaceutically acceptable salt thereof is used in combination with the immunosuppressive therapy.

In some embodiments, the present disclosure provides methods of using Compound (I) or a pharmaceutically acceptable salt thereof as a replacement therapy for a corticosteroid therapy for a disease chosen from membranous nephropathy (MN), IgG4-related diseases, and antiphospholipid syndrome (APS). In some embodiments, the present disclosure provides methods of using Compound (1) or a pharmaceutically acceptable salt thereof as a replacement therapy for a disease chosen from membranous nephropathy (MN), IgG4-related diseases, and antiphospholipid syndrome (APS), wherein a corticosteroid is used as a first or second line therapy for the disease. In some embodiments, Compound (1) or a pharmaceutically acceptable salt thereof is used in place of the corticosteroid. In some embodiments, Compound (I) or a pharmaceutically acceptable salt thereof is used in combination with the corticosteroid. In some embodiments, the present disclosure provides methods of using Compound (I) or a pharmaceutically acceptable salt thereof as a replacement therapy for a disease chosen from membranous nephropathy (MN), IgG4-related diseases, and antiphospholipid syndrome (APS), wherein a corticosteroid is used as a first or second line maintenance therapy for the disease. In some embodiments, Compound (I) or a pharmaceutically acceptable salt thereof is used in place of the corticosteroid. In some embodiments, Compound (I) or a pharmaceutically acceptable salt thereof is used in combination with the corticosteroid. In some embodiments, the corticosteroid is a glucocorticoid.

In some embodiments, the present disclosure provides methods of eliminating or reducing a therapeutic dose of a corticosteroid therapy used in chronic maintenance therapy in the treatment of a disease chosen from membranous nephropathy (MN), IgG4-related diseases, and antiphospholipid syndrome (APS) in a mammal in need thereof, wherein the corticosteroid therapy is used as a first or second line treatment, comprising administering to said mammal in need of said treatment a therapeutically effective amount of Compound (1) or a pharmaceutically acceptable salt thereof. In some embodiments, Compound (I) or a pharmaceutically acceptable salt thereof is used in place of the corticosteroid therapy. In some embodiments, Compound (I) or a pharmaceutically acceptable salt thereof is used in combination with the corticosteroid therapy. In some embodiments, the corticosteroid therapy is a glucocorticoid.

In some embodiments of the present disclosure, Compound (I) or a pharmaceutically acceptable salt thereof is administered in combination with a noncorticosteroidal immunosuppressive and/or anti-inflammatory agent.

In some embodiments of the present disclosure. Compound (I) or a pharmaceutically acceptable salt thereof is administered in combination with an active pharmaceutical ingredient chosen from interferon alpha, interferon gamma, cyclophosphamide, tacrolimus, mycophenolate mofetil, methotrexate, dapsone, sulfasalazine, azathioprine, an anti-CD20 agent (*e.g*., rituximab, dupilumab, ofatumumab, obinutuzumab, or veltuzumab, or a biosimilar version of any of the foregoing), an anti-TNF alpha agent (*e.g*., etanercept, infliximab, golimumab, adalimumab, or certolizumab pegol, or a biosimilar version of any of the foregoing), an anti-lL-4 agent toward ligand or its receptors (*e.g*., dupilumab or a biosimilar version thereof), an anti-IL-6 agent toward ligand or its receptors (*e.g.,* tocilizumab. sarilumab. olokizumab, elsililumab, or siltuximab. or a biosimilar version of any of the foregoing). an anti-IL-17 agent to ligand or its receptors (*e.g.,* secukinumab. ustekinumab, brodalumab, or ixekizumab, or a biosimilar version of any of the foregoing), an anti-IL-23 agent toward ligand or its receptors (*e.g*., guselkumab or risankizumab, or a biosimilar version of either), an anti-IL-12/23 agent toward ligand or receptors (*e.g*., ustekinumab or a biosimilar version thereof), an anti-IL-12 agent toward ligand or its receptors, an anti-IL-1 agent to ligand or its receptors (*e.g*., rilonacept, canakinumab. or anakinra, or a biosimilar version of any of the foregoing), an anti-IL-2 agent to ligand or its receptors (*e.g*., basiliximab or daclizumab, or a biosimilar version of either), an anti-CD2 agent (*e.g*., alefacept or a biosimilar version thereof), an anti-CD3 agent (*e.g*., muromonab-cd3 or a biosimilar version thereof), an anti-CD80/86 agent *(e.g.,* abatacept or belatacept, or a biosimilar version of either). an anti-sphingosine-1-phosphate receptor agent (*e.g.,* fingolimod or a biosimilar version thereof), an anti-C5 agent (*e.g.*, eculizumab or a biosimilar version thereof), an anti-integrin alpha4 agent (*e.g*., natalizumab or a biosimilar version thereof), an anti-αβ₇ agent (*e.g*., vedolizumab or a biosimilar version thereof), an anti-mTOR agent (*e.g.,* sirolimus or everolimus), an anti-calcineurin agent (*e.g.,* tacrolimus), an anti-BAFF/BlyS agent (*e.g.*, belimumab, VAY736, or blisibimod, or a biosimilar version of any of the foregoing), leflunomide, and teriflunomide.

### Embodiments:

Non-limiting embodiments of this disclosure include:
1. A method of treating a disease chosen from membranous nephropathy (MN), an IgG4-related disease (IgG4-RD), and antiphospholipid syndrome (APS) in a mammal, comprising administering to said mammal a pharmaceutical composition comprising:
   a compound chosen from (E) isomer, (Z) isomer, and a mixture of (E) and (Z) isomers of 2-[(3R)-3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitnle; and/or
   a pharmaceutically acceptable salt of any of the foregoing compounds; and
   a pharmaceutically acceptable carrier or excipient.
2. The method of Embodiment 1, wherein the disease is acute.
3. The method of Embodiment 1 or 2, wherein the disease is an IgG4-RD disease flare.
4. The method of any one of Embodiments 1-3, wherein the pharmaceutical composition is orally administered.
5. The method of any one of Embodiments 1-4, wherein the pharmaceutical composition is administered daily.
6. The method of any one of Embodiments 1-5, wherein the pharmaceutical composition is orally administered daily.
7. The method of any one of Embodiments 1-6, wherein the pharmaceutical composition is administered in place of immunosuppressive therapy.
8. The method of any one of Embodiments 1-7, wherein the pharmaceutical composition is administered in place of corticosteroid therapy.
9. The method of any one of Embodiments 1-6, wherein the pharmaceutical composition is administered in combination with immunosuppressive therapy.
10. The method of any one of Embodiments 1-6. wherein the pharmaceutical composition is administered in combination with corticosteroid therapy.
11. The method of any of Embodiments 1-6 or 8-10, wherein the pharmaceutical composition is administered in combination with a noncorticosteroidal immunosuppressive and/or anti-inflammatory agent.
12. The method of any one of Embodiments 1 or 4-6, wherein the pharmaceutical composition is administered in place of immunosuppressive maintenance therapy.
13. The method of any one of Embodiments 1 or 4-6, wherein the pharmaceutical composition is administered in place of corticosteroid maintenance therapy.
14. The method of any one of Embodiments 1, 2, or 4-6, wherein the pharmaceutical composition is administered in combination with immunosuppressive maintenance therapy.
15. The method of any one of Embodiments 1, 2, or 4-6, wherein the pharmaceutical composition is administered in combination with corticosteroid maintenance therapy.
16. The method of any one of Embodiments 13-15, wherein the pharmaceutical composition is administered in combination with a noncorticosteroidal immunosuppressive and/or anti-inflammatory agent.
17. The method of any one of Embodiments 1-16, wherein the pharmaceutical composition comprises a compound which is a substantially pure (E) or (Z) isomer of 2-[(3R)-3-[4-amino-3-(2-fluoro-4-phenoxy-phenyl)pyrazolo[3.4-d]pyrimidin-1-yl]piperidine-1-carbonyl]-4-methyl-4-[4-(oxetan-3-yl)piperazin-1-yl]pent-2-enenitrile, and/or
   a pharmaceutically acceptable salt of said compound; and
   a pharmaceutically acceptable carrier or excipient.
18. The method of any one of Embodiments 1-17, wherein at least about 85% w/w of 2-[(3R)-3-[4-amino-3-(2-fluoro-4-phenoxy-phenyl)pyrazolo[3,4-d]pyrimidin-1-yl]piperidine-1-carbonyl]-4-methyl-4-(oxetan-3-yl)piperazin-1-yl]pent-2-enenitrile or at least about 85% w/w of a pharmaceutically acceptable salt of 2-[(3R)-3-|4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile is the (E) isomer.
19. The method of any one of Embodiments 1-18, wherein the mammal is a human.
20. The method of any one of Embodiments 1-19, wherein the IgG4-related disease is characterized by an IgG4-RD Responder Index (RI) greater than or equal to 2 in at least one organ system.
21. The method of any one of Embodiments 1-20, wherein the IgG4-related disease is characterized by a serum IgG4 concentration greater than 1.5 times the upper limit of normal.
22. The method of any one of Embodiments 1-21, wherein the IgG4-related disease is chosen from IgG4-related sialadenitis. IgG4-related ophthalmic disease (IgG4-ROD). IgG4-related pharyngitis, IgG4-related thyroid disease, IgG4-related hypophysitis, IgG4-related pachymeningitis, IgG4-related leptomeningitis, IgG4-related pancreatitis, IgG4-related lung disease, IgG4-related pleuritis, IgG4-related hepatopathy, IgG4-related sclerosing cholangitis. IgG4-related cholecystitis, IgG4-related aortitis, IgG4-related periaortitis, IgG4-related periarteritis, IgG4-related pericarditis, IgG4-related mediastinitis, IgG4-related retroperitoneal fibrosis, IgG4-related mesenteritis, IgG4-related mastitis, IgG4-related kidney disease (IgG4-RKD). IgG4-related prostatitis, IgG4-related perivasal fibrosis, IgG4-related paratesticular pseudotumor, IgG4-related epididy mo-orchitis, IgG4-related lymphadenopathy, IgG4-related skin disease, and IgG4-related perineural disease, or flares of any of the preceding IgG4-related diseases.
23. The method of Embodiment 1, wherein the pharmaceutical composition is administered in combination with an active pharmaceutical ingredient chosen from interferon alpha, interferon gamma, cyclophosphamide, tacrolimus, mycophenolate mofetil, methotrexate, dapsone, sulfasalazine, azathioprine, an anti-CD20 agent, an anti-TNF alpha agent, an anti-IL6 agent toward ligand or its receptors, an anti-IL17 agent to ligand or its receptors, an anti-IL1 agent to ligand or its receptors, an anti-IL2 agent to ligand or its receptors, an anti-CD2 agent, an anti-CD3 agent, an anti-CD80/86 agent, an anti-sphingosine-1-phosphate receptor agent, an anti-C5 agent, an anti-mTOR agent, an anti-calcineurin agent, an anti-BAFF/BlyS agent, leflunomide, and teriflunomide.
24. The method of Embodiment 1, wherein the pharmaceutical composition is administered in combination with rituximab, ofatumumab, obinutuzumab, or veltuzumab, or a biosimilar version of any of the foregoing.
25. The method of any one of Embodiments 1-6, 9-11. or 14-24, wherein the mammal is treatment naïve with respect to a immunosuppressive therapy.
26. The method of any one of Embodiments 1-6, 9-11, or 14-24, wherein the mammal is treatment naïve with respect to any immunosuppressive therapy.
27. The method of any one of Embodiments 1-24, wherein the mammal has been previously treated with an immunosuppressive therapy.

### BRIEF DESCRIPTION OF THE DRAWINGS

FIG. 1 illustrates the study design for investigating dose dependent inhibition in an anti-GBM (anti-glomerular basement membrane) mouse glomerulonephritis model with Compound (I) (PRN1008) treatment. The mouse anti-GBM glomerulonephritis model involves antibody mediated autoimmunity, and the model is histologically and mechanistically similar to glomerulonephritis in humans. The model also includes kidney deposition of immune complexes (IC), targeting glomerular basement membrane.
FIG. 2 shows dose dependent inhibition of serum blood urea nitrogen (BUN) levels with Compound (I) (PRN1008) treatment in a mouse anti-GBM glomerulonephritis model. BUN levels provide a measure of kidney function.
FIGs. 3A and 3B shows dose dependent inhibition of severe proteinuria with Compound (I) (PRN 1008) treatment in a mouse anti-GBM glomerulonephritis model.
FIG. 4 shows reduced proteinuria with Compound (I) (PRN1008) treatment in a mouse anti-GBM glomerulonephritis model.
FIG. 5 shows dose dependent inhibition of kidney weight gain with Compound (I) (PRN1008) treatment in a mouse anti-GBM glomerulonephritis model. Kidney weight gain is a surrogate for kidney inflammation.
FIG. 6 shows that Compound (1) (PRN1008) significantly reduced kidney pathology, superior to a steroid comparator (Dex), in a mouse anti-GBM glomerulonephritis model.

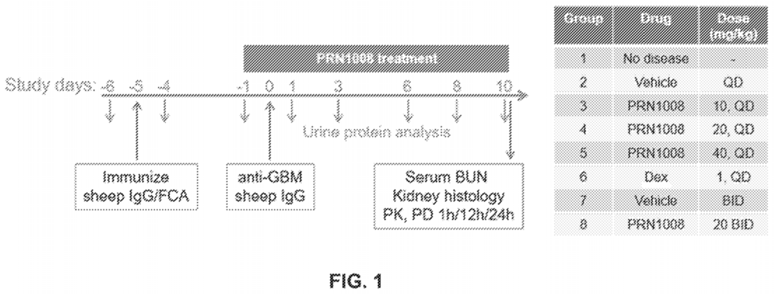

In FIGs. 1-6, Dex refers to dexamethasone, a potent synthetic member of the glucocorticoid class of steroid hormones.

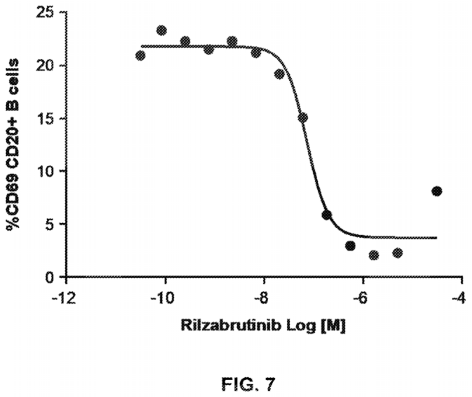

FIG. 7 shows inhibition of B cell activation as measured by anti-IgM-induced CD69 expression on CD20+ B-cells from human whole blood treated with increasing concentrations of Compound (I) (rilzabrutinib), as measured by flow cytometry (n = 4).

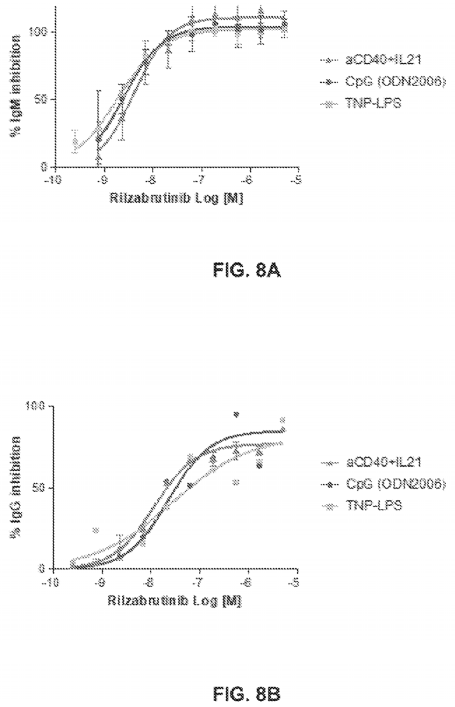

FIGs. 8A and 8B show inhibition of IgG and IgM production by Compound (I) (rilzabrutinib). Rilzabrutinib-treated enriched B cells were stimulated with CpG (n = 4), TNP-LPS (n = 4-6) or anti-CD40+ IL-21 (n = 2-7) for 7 days. Measurement of IgG and IgM showed that both T-dependent and T-independent antibody production was inhibited by rilzabrutinib. Curves depict the results of representative experiments.

### Definitions:

As used herein, "a" or "an" entity refers to one or more of that entity, *e.g*., "a compound" refers to one or more compounds or at least one compound unless stated otherwise. As such, the terms "a" (or "an"), "one or more", and "at least one" are used interchangeably herein.

As used herein, the term "about" means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%.

As used herein, "Compound **(I)"** refers to the (E) isomer, (Z) isomer, or a mixture of (E) and (Z) isomers of 2-[(3R)-3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile, which has the following structure: 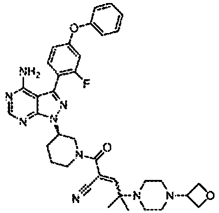 or a pharmaceutically acceptable salt thereof. The 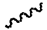 line at the alkene carbon in Compound (I) denotes that Compound (I) or a pharmaceutically acceptable salt thereof can be (E) isomer, (Z) isomer, or a mixture of (E) and (Z) isomers.

All polymorphic forms and hydrates of Compound (1) are within the scope of this disclosure and claims appended hereto.

It will be understood by a person of ordinary skill in the art that when a compound is denoted as the (R) isomer, the compound may contain the corresponding (S) stereoisomer as an impurity, *i.e*., the (S) stereoisomer in less than about 1% by wt and vice versa.

As used herein, "substantially pure" in connection with a geometric or isomeric form refers to a compound, such as Compound (I), wherein more than 70% by weight of the compound is present as the given isomeric form. For example, the phrase "the solid form of Compound (I) is a substantially pure (E) isomer of Compound (1)" refers to the solid form of Compound (I) having at least 70% by weight of the solid form of Compound (I) being in the (E) isomeric form, and the phrase "the solid form of Compound (I) is a substantially pure (Z) isomer of Compound (I)" refers to the solid form of Compound (I) having at least 70% by weight of the solid form of Compound (I) being in the (Z) isomeric form. In some embodiments, at least 80% by weight of the solid form of Compound (1) is the (E) form or at least 80% by weight of the solid form of Compound (I) is the (Z) form. In some embodiments, at least 85% by weight of the solid form of Compound (I) is in the (E) form or at least 85% by weight of the solid form of Compound (I) is in the (Z) form. In some embodiments, at least 90% by weight of the solid form of Compound (1) is in the (E) form or at least 90% by weight of the solid form of Compound (I) is in the (Z) form. In some embodiments, at least 95% by weight of the solid form of Compound (1) is in the (E) form or at least 95% by weight of the solid form of Compound (I) is in the (Z) form. In some embodiments, at least 97% by weight, or 98% by weight, of the solid form of Compound (I) is in the (E) form or at least 97% by weight, or 98% by weight, of the solid form of Compound (I) is in the (Z) form. In some embodiments. at least 99% by weight of the solid form of Compound (I) is in the (E) form or at least 99% by weight of the solid form of Compound (I) is in the (Z) form. The relative amounts of (E) and (Z) isomers in a solid mixture can be determined according to standard methods and techniques known in the art.

"Acute," as used herein, means a disease with a rapid onset and/or a short course (e.g., a disease flare).

As used herein, a "pharmaceutically acceptable salt" of a compound means a salt that is pharmaceutically acceptable and that possesses the desired pharmacological activity of the parent compound. Such salts include, but are not limited to:
acid addition salts, formed with inorganic acids such as hydrochloric acid, hydrobromic acid, sulfuric acid, nitric acid, phosphoric acid, and the like; or formed with organic acids such as formic acid, acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or
salts formed when an acidic proton present in the parent compound either is replaced by a metal ion, *e.g*., an alkali metal ion, an alkaline earth ion, or an aluminum ion; or coordinates with an organic base such as ethanolamine, diethanolamine, triethanolamine, tromethamine, *N*-methylglucamine, and the like. It is understood that the pharmaceutically acceptable salts are non-toxic.

Additional information on suitable pharmaceutically acceptable salts can be found in Remington's Pharmaceutical Sciences, 17th ed., Mack Publishing Company, Easton, PA, 1985. portions of which relate to suitable pharmaceutically acceptable salts are incorporated herein by reference. *See also* Berge at al., Pharmaceutical Salts, Journal of Pharmaceutical Sciences, 1, Volume 66, Number 1, January 1997.

Treatment decisions often follow formal or informal algorithmic guidelines. Treatment options can often be ranked or prioritized into lines of therapy: first-line therapy, second-line therapy, third-line therapy, and so on. First-line therapy is the first therapy that will be tried. Its priority over other options is usually either (I) formally recommended on the basis of clinical trial evidence for its best-available combination of efficacy, safety, and tolerability or (2) chosen based on the clinical experience of the physician. If a first-line therapy either fails to resolve the issue or produces intolerable side effects, additional (second-line) therapies may be substituted or added to the treatment regimen, followed by third-line therapies, and so on. Accordingly, "first-line" therapy as used herein means therapy usually given when someone is diagnosed with a particular disease or condition and can be categorized as standard of care.

"Maintenance therapy," as used herein, means a therapy, therapeutic regimen, or course of therapy which is administered subsequent to an initial course of therapy administered to a patient with a disease. Maintenance therapy can be used to halt, slow down, or even reverse the progression of the disease, to maintain the improvement in health achieved by the initial treatment and/or enhance the gains achieved by the initial therapy.

A "pharmaceutically acceptable carrier or excipient" means a carrier or an excipient that is useful in preparing a pharmaceutical composition that is generally safe, non-toxic, and neither biologically nor otherwise undesirable, and includes a carrier or an excipient that is acceptable for veterinary use as well as human pharmaceutical use. As used herein, "a pharmaceutically acceptable carrier or excipient" means one or more pharmaceutically acceptable carriers or excipients.

As used herein. "treating," "treat," or "treatment" of a disease includes:
(1) inhibiting the disease, *i.e.,* arresting or reducing the development of the disease or its clinical symptoms; or
(2) relieving the disease, *i.e.,* causing regression of the disease or its clinical symptoms.

As used herein, a "therapeutically effective amount" means the amount of a compound of the present disclosure that, when administered to a mammal, *e.g*., a human, for treating a disease, is sufficient to effect such treatment for the disease. The "therapeutically effective amount" will vary depending on the compound, the disease and its severity and the age, weight, etc., of the mammal to be treated.

As used herein. "QD" means once a day.

As used herein, "*BID" means twice a day.

"Mammal," as used herein, means animals such as dogs, cats, and humans.

"Membranous nephropathy (MN)" is a kidney disease that affects the filters (glomeruli) of the kidney and can cause protein in the urine, as well as decreased kidney function and swelling. It is also known as membranous glomerulopathy and glomerulonephritis. While not wishing to be bound by theory, the treatment of MN is believed to be mediated by autoantibody depletion and inhibition of platelet aggregation. Accordingly, BTK inhibitors, such as Compound (I), may be useful in the treatment of MN.

"IgG4-related disease (IgG4-RD)" is a fibro-inflammatory condition that can affect nearly any organ system. If left untreated. IgG4-RD can lead to sever morbidity and mortality, including organ dysfunction and organ failure. IgG4-RD is characterized by tissue infiltration with lymphocytes and IgG4-secreting plasma cells, various degrees of fibrosis (scarring), and a usually prompt response to oral steroids. Common presentations include major salivary and lacrimal gland enlargement, orbital disease, autoimmune pancreatitis, retroperitoneal fibrosis, and tubulointerstitial nephritis. In some embodiments, IgG4-RD is characterized by an IgG4-RD Responder Index (RI) greater than or equal to 2 in at least one organ system. In some embodiments, IgG4-RD is characterized by a serum IgG4 concentration greater than 1.5 times the upper limit of normal. In some embodiments, IgG4-RD is characterized by an IgG4-RD Responder Index (RI) greater than or equal to 2 in at least one organ system and a serum IgG4 concentration greater than 1.5 times the upper limit of normal.

There are no approved drugs for the treatment of IgG4-RD. Treatment is typically corticosteroids (CS); however, patients often relapse after CS are tapered and thus require chronic CS dosing, which leads to severe and debilitating side effects. Rituximab has been shown to have an effective clinical response; however, patients frequently relapse after treatment as well. While not wishing to be bound by theory, it is believed that multiple mechanisms of action can potentially lead to positive outcomes in IgG4-RD by impacting many of the driving features of the disease, including inflammation, allergic components (IgE and eosinophils), monocytes and macrophages (involved in fibrosis), and B cells implicated in initiation and maintenance of disease. Accordingly, BTK inhibitors, such as Compound (I), may be useful in the treatment of IgG4-RD.

When the organ implicated is the salivary gland, the IgG4-related disease is called IgG4-related sialadenitis. Non-limiting examples of IgG4-related sialadenitis include IgG4-related submandibular gland disease and IgG4-related parotitis.

When the organ implicated is the orbit, the IgG4-related disease is called IgG4-related ophthalmic disease (IgG4-ROD). Non-limiting examples of IgG4-ROD include IgG4-related dacryoadenitis (lacrimal glands), IgG4-related orbital inflammation (or IgG4-related orbital inflammatory pseudotumor), IgG4-related orbital myositis (extraocular muscles), and IgG4-related pan-orbital inflammation.

When the organ implicated is paranasal sinuses, non-limiting examples of IgG4-related disease include chronic sinusitis and eosinophilic angiocentric fibrosis (upper respiratory tract and orbit).

When the organ implicated is the pharynx, a non-limiting example of IgG4-related disease is IgG4-related pharyngitis.

When the organ implicated is the thyroid gland, a non-limiting example of IgG4-related disease is IgG4-related thyroid disease.

When the organs implicated are soft tissues of the head and neck, non-limiting examples of IgG4-related diseases include idiopathic cervical fibrosis, sclerosing cervicitis, and cervical fibrosclerosis.

When the organ implicated is the pituitary gland, the IgG4-related disease is called IgG4-related hypophysitis. Non-limiting examples include IgG4-related panhypophysitis (all of pituitary gland), IgG4-related adenohypophysitis (anterior pituitary), and IgG4-related infundibuloneurohypophysitis (posterior pituitary and pituitary stalk).

When the organs implicated are the meninges, non-limiting examples of IgG4-related disease include idiopathic hypertrophic pachymeningitis.

When the organ implicated is the pancreas, non-limiting examples of IgG4-related disease include Type 1 autoimmune pancreatitis, lymphoplasmacytic sclerosing pancreatitis, and chronic pancreatitis with diffuse irregular narrowing of the main pancreatic duct.

When the organ implicated is the lung, non-limiting examples of IgG4-related disease include pulmonary inflammatory pseudotumor.

When the organ implicated is the pleura, non-limiting examples of IgG4-related disease include pleuritis.

When the organ implicated is the liver, non-limiting examples of IgG4-related disease include IgG4-relaed hepatopathy and IgG4-related cholecystitis.

When the organ implicated is the bile duct, non-limiting examples of IgG4-related disease include IgG4-related sclerosing cholangitis.

When the organ implicated is the gall bladder, non-limiting examples of IgG4-related disease include IgG4-related cholecystitis.

When the organ implicated is the aorta, non-limiting examples of IgG4-related disease include IgG4-related aortitis and IgG4-related periaortitis.

When the organ implicated is branches of the aorta (including coronary, renal, or iliac arteries), non-limiting examples of IgG4-related disease include IgG4-related periarteritis.

When the organ implicated is the breast, non-limiting examples of IgG4-related disease include IgG4-related mastitis.

When the organ implicated is the kidney, the IgG4-related disease is called IgG4-related kidney disease (IgG4-RKD). Non-limiting examples of IgG4-KD include IgG4-related tubulointerstitial nephritis (IgG4-TIN), membranous glomerulonephritis secondary to IgG4-related disease, and IgG4-related renal pyelitis (renal pelvis).

When the organ implicated is the prostrate, non-limiting examples of IgG4-related disease include IgG4-related prostatitis.

When the organ implicated is Vas deferens, non-limiting examples of IgG4-related disease include IgG4-related perivasal fibrosis.

When the organ implicated is the scrotum, non-limiting examples of IgG4-related disease include IgG4-related paratesticular pseudotumor and IgG4-related epididymo-orchitis.

When the organs implicated are the lymph nodes, non-limiting examples of IgG4-related disease include IgG4-related lymphadenopathy.

When the organ implicated is the skin, non-limiting examples of IgG4-related disease include IgG4-related skin disease, *e.g*., angiolymphoid hyperplasia with eosinophilia and cutaneous pseudolymphoma.

When the organ implicated is the nerve, non-limiting examples of IgG4-related disease include IgG4-related perineural disease.

In some embodiments of the present disclosure, the IgG4-related disease is acute (such as, *e.g*., an IgG4-related disease flare).

"Antiphospholipid syndrome (APS)," sometimes known as Hughes syndrome, is a disorder of the immune system that causes an increased risk of blood clots. As such, people with APS are at greater risk of developing conditions such as deep vein thrombosis (DVT) (a blood clot that usually develops in the legs), arterial thrombosis (an artery clot) having the potential to cause stroke or heart attack, and blood clots in the brain which can lead to issues with mobility, speech, vision and memory. While not wishing to be bound by theory, auto-antibodies produced by plasma cells are believed to be important in the pathology of APS. Thrombotic events and pregnancy morbidity are also mediated by immune infiltration. Accordingly, BTK inhibitors, such as Compound (I), may be useful in the treatment of APS.

### Formulations and Administration:

In general, the compounds of this disclosure will be administered in a therapeutically effective amount by any of the accepted modes of administration (*e.g*., oral administration) for agents that serve similar utilities. Therapeutically effective amounts of compounds of this disclosure may range from about 0.01 to about 500 mg per kg patient body weight per day, which can be administered in single or multiple doses. A suitable dosage level may be from about 0.1 to about 250 mg/kg per day, such as about 0.5 to about 100 mg/kg per day.

A suitable dosage level may also be about 0.01 to about 250 mg/kg per day, such as about 0.05 to about 100 mg/kg per day, and further such as about 0.1 to about 50 mg/kg per day. Within this range, the dosage can be about 0.05 to about 0.5, such as about 0.5 to about 5, and further such as about 5 to about 50 mg/kg per day. For oral administration, the compositions can be provided in the form of tablets containing about 1 to about 1000 milligrams of the active ingredient, particularly about 1, 5, 10, 15, 20, 25, 50, 75, 100, 150, 200, 250, 300, 400, 500, 600, 750, 800, 900. and 1000 milligrams of the active ingredient. The actual amount to be administered of the compound of this disclosure, *i.e.,* the active ingredient, will depend upon numerous factors such as the severity of the disease to be treated, the age and relative health of the patient, the potency of the compound being utilized, the route and form of administration, and other factors.

In general, compounds of this disclosure will be administered as pharmaceutical compositions by any one of the following routes: oral, systemic (*e.g.,* transdermal, intranasal or by suppository), or parenteral (*e.g*., intramuscular, intravenous, or subcutaneous) administration. In some embodiments, pharmaceutical compositions will be administered orally using a convenient daily dosage regimen, which can be adjusted according to the degree of affliction. Compositions can take the form of tablets, pills, capsules, semisolids, powders, sustained release formulations, solutions, suspensions, elixirs. aerosols, or any other appropriate compositions.

The choice of formulation depends on various factors such as the mode of drug administration (*e.g*., for oral administration, formulations in the form of tablets, pills or capsules, including enteric coated or delayed release tablets, pills or capsules) and the bioavailability of the drug substance. Recently, pharmaceutical formulations have been developed especially for drugs that show poor bioavailability based upon the principle that bioavailability can be increased by increasing the surface area, *i*.*e*., by decreasing particle size. For example, U.S. Patent No. 4,107.288 describes a pharmaceutical formulation having particles in the size range from 10 to 1,000 nm in which the active material is supported on a cross-linked matrix of macromolecules. U.S. Patent No. 5,145,684 describes the production of a pharmaceutical formulation in which the drug substance is pulverized to nanoparticles (average particle size of 400 nm) in the presence of a surface modifier and then dispersed in a liquid medium to give a pharmaceutical formulation that exhibits remarkably high bioavailability. The disclosures of those two patents are incorporated by reference with respect to those portions relating to pharmaceutical formulations.

The compositions comprise, in general, a compound of this disclosure in combination with a pharmaceutically acceptable excipient. Pharmaceutically acceptable excipients are non-toxic, aid administration, and do not adversely affect the therapeutic benefit of the compound of this disclosure. Such excipient may be any solid, liquid, semi-solid or, in the case of an aerosol composition, gaseous excipient that is generally available to one of skill in the art.

Solid pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk and the like. Liquid and semisolid excipients may be chosen from glycerol, propylene glycol, water, ethanol and various oils, including those of petroleum, animal, vegetable or synthetic origin, *e.g*., peanut oil, soybean oil, mineral oil, sesame oil, etc. Liquid carriers. *e.g.,* for injectable solutions, include water, saline, aqueous dextrose, and glycols.

Compressed gases may be used to disperse a compound of this disclosure in aerosol form. Inert gases suitable for this purpose are nitrogen, carbon dioxide, etc.

Other suitable pharmaceutical excipients and their formulations are described in Remington's Pharmaceutical Sciences, edited by E. W. Martin (Mack Publishing Company, 20th ed., 2000), incorporated by reference herein with respect to the portions relating to pharmaceutical excipients and their formulations.

The level of the compound in a formulation can vary within the full range employed by those skilled in the art. Typically, the formulation will contain, on a weight percent (wt. %) basis, from about 0.01-99.99 wt. % of a compound of this disclosure based on the total formulation, with the balance being a suitable pharmaceutical excipients. For example, the compound is present at a level of about 1-80 wt. %. With respect to the numerical range 0.01-99.99 "about" denotes less than 0.01%. With respect to the numerical range 1 to 80, "about" denotes 0.05 with respect to 1 and 10 with respect to 80, thus covering a range from 0.05 to 90 wt. %.

The compounds of this disclosure may be used in combination with one or more other drugs in the treatment of diseases or conditions for which compounds of this disclosure or the other drugs may have utility. Such other drug(s) may be administered, by a route and in an amount commonly used therefore, contemporaneously, such as fixed dose combination, or sequentially with a compound of the present disclosure. When a compound of this disclosure is used contemporaneously with one or more other drugs, a pharmaceutical composition in unit dosage form containing such other drugs and the compound of the present disclosure, *i.e.,* a fixed dose compound, is preferred. However, the combination therapy may also include therapies in which the compound of this disclosure and one or more other drugs are administered on different overlapping schedules or even non-overlapping schedules. It is also contemplated that situations will arise that when used in combination with one or more other active ingredients, the compounds of the present disclosure and the other active ingredients may be used in lower doses than when each is used singly.

All publications and patents mentioned herein are hereby incorporated by reference in their entirety as if each individual publication or patent was specifically and individually indicated to be incorporated by reference.

Claims or descriptions that include "or" or "and/or" between at least one members of a group are considered satisfied if one, more than one, or all of the group members are present in, employed in, or otherwise relevant to a given product or process unless indicated to the contrary or otherwise evident from the context. The disclosure includes embodiments in which exactly one member of the group is present in, employed in, or otherwise relevant to a given product or process. The disclosure includes embodiments in which more than one, or all the group members are present in, employed in, or otherwise relevant to a given product or process.

Furthermore, the disclosure encompasses all variations, combinations, and permutations in which at least one limitation, element, clause, and descriptive term from at least one of the listed claims is introduced into another claim. For example, any claim that is dependent on another claim can be modified to include at least one limitation found in any other claim that is dependent on the same base claim. Where elements are presented as lists, e.g., in Markush group format, each subgroup of the elements is also disclosed, and any element(s) can be removed from the group. It should be understood that, in general, where the disclosure, or aspects of the disclosure, is/are referred to as comprising particular elements and/or features, embodiments of the disclosure or aspects of the disclosure consist, or consist essentially of, such elements and/or features. For purposes of simplicity, those embodiments have not been specifically set forth in haec verba herein. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the disclosure, to the tenth of the unit of the lower limit of the range, unless the context clearly dictates otherwise.

Those of ordinary skill in the art will recognize or be able to ascertain using no more than routine experimentation, many equivalents to the specific embodiments of the disclosure described herein. Such equivalents are intended to be encompassed by the following claims.

### EXAMPLES

The following examples are intended to be illustrative and are not meant in any way to limit the scope of the disclosure.

### Example 1: Mouse anti-GBM glomerulonephritis model

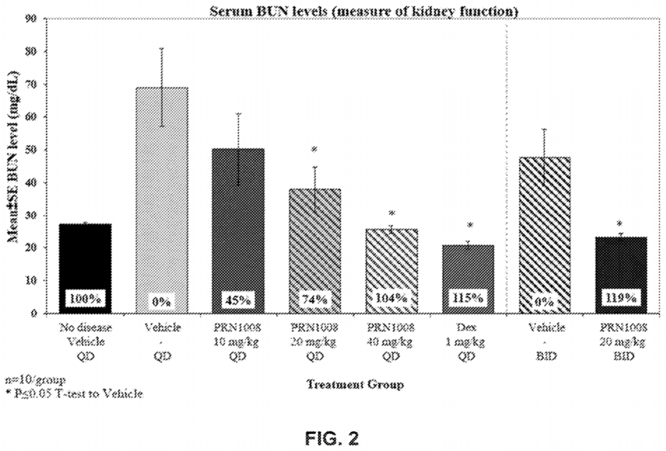

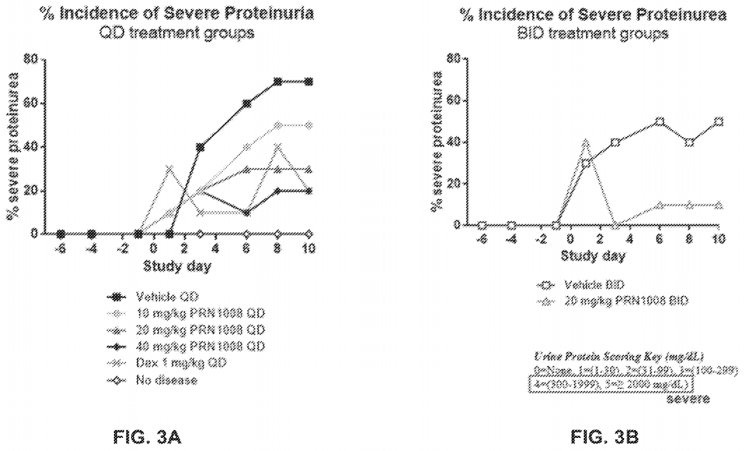

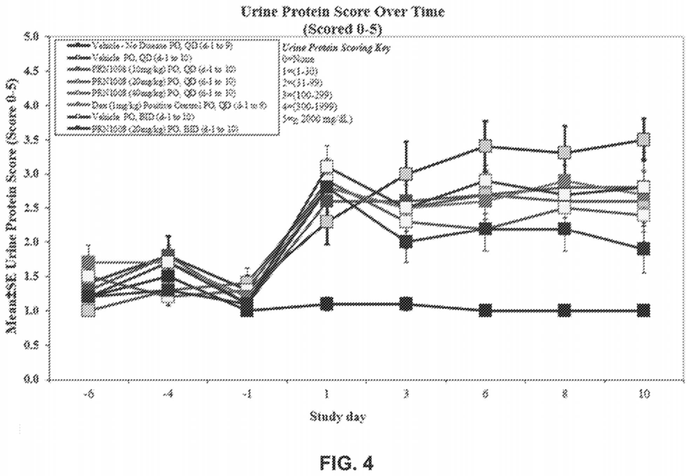

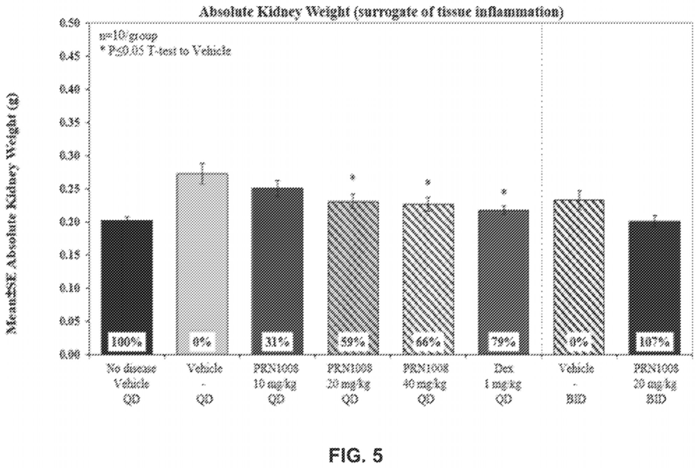

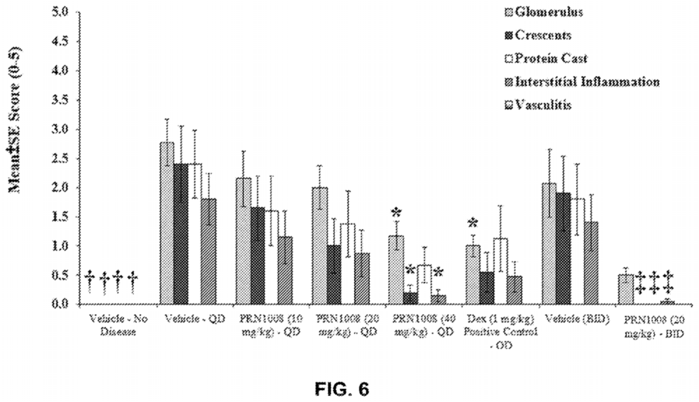

The efficacy of Compound (I) (also referred to as PRN 1008) relative to the corticosteroid dexamethasone (Dex) was tested in a mouse anti-GBM glomerulonephritis model according to the design shown in FIG. 1. Briefly, to induce glomerulonephritis, mice were pre-sensitized with sheep IgG/FCA (Study Day -5). Five days later (Study Day 0), the mice received anti-GBM sheep IgG. Treatment with vehicle, Compound (1), or Dex with various dosage regimes (Compound (I): 10 mg/kg, 20 mg/kg, or 40 mg/kg QD or 20 mg/kg BID; Dex: 1 mg/kg QD; Vehicle: QD or BID) began at Study Day -1, one day prior to injection with anti-GBM sheep IgG. Treatment continued until Study Day 10, for eleven days treatment in total. Urine protein analysis was conducted on Study Days -6, -4, -1, 1, 3, 6, 8, and 10. Following Study Day 10, mice serum BUN levels were analyzed as a measure of kidney function, and kidney histology was performed. In the mouse anti-GBM glomerulonephritis model, dose dependent inhibition of serum BUN levels (FIG. 2), severe proteinuria (FIG. 3), and kidney weight gain (a surrogate for kidney inflammation) (FIG. 5) were observed. Additionally, treatment with Compound (I) led to reduced proteinuria during the study (FIG. 4), and Compound (I) reduced kidney pathology (FIG. 6), providing favorable results relative to Dex.

### Example 2: Open-label, 2-arm study to assess the safety and efficacy of Compound (I) in IgG4-RD patients who are refractory to rituximab

To assess the safety and efficacy of daily oral administration of Compound (I) in IgG4-RD patients who are refractory to rituximab, patients are being enrolled in an open-label, 2-arm study. Patients with an IgG4-RD Responder Index (RI) greater than or equal to 2 at screening (*i.e.,* up to four days before the start of treatment) in at least one organ system and optionally additionally with a serum IgG4 concentration greater than 1.5 times the upper limit of normal will be treated with Compound (I) for 12 to 52 weeks. After 12 weeks, the safety and ability/efficacy of oral daily administration of Compound (I) to induce glucocorticoid-free disease remission in IgG4-RD will be evaluated. Complete remission will be defined as an IgG4-RD RI score of zero at week 12 and no use of glucocorticoids or other immunosuppressive medications between weeks 4 and 12. After extended treatment of up to 52 weeks, the effects on Compound (I) on IgG4-RD disease flare and glucocorticoid sparing will be evaluated, along with the effect of Compound (I) on changes in serum concentrations of IgG4, IgE, IgG, IgM, C3, C4, and other serological parameters over time.

### Example 3: Assay method for inhibition of B-cell antibody production by Compound (I)

B-cells were enriched from leukocyte concentrate of TrimaAccel^{®} LRS chamber recovered after a plateletpheresis procedure from healthy volunteers (Stanford Blood Center, Palo Alto, CA) using the Straight from LRSC CD19 Microbead Kit (Miltenyi Biotech). B-cells were treated with 10 concentrations of 3-fold serial dilutions rilzabrutinib in DMSO for 1 hour at 37°C, 5% CO₂. Compound (I) (rilzabrutinib)-treated B-cells and DMSO control were then stimulated with trinitrophenyl conjuaged to lipopolysaccharide (TNP-LPS (Santa Cruz Biotechnology)), 5'-Cytosine-phosphate-Guanine-3'(CpG (ODN2006, Invivogen)), or anti-CD40 (BD Biosciences) + IL21 (R&D Systems) for 7 days at 37°C, 5% CO₂. Compound (I) inhibition of antibody production was determined by measurement of IgG and IgM in the cell culture lysate using human IgG and IgM AlphaLISA kits (Perkin Elmer). IC₅₀ was calculated in GraphPad Prism with non-linear regression using "log (inhibitor) vs. response - Variable slope (four parameters)" analysis with no constraints.

### Example 4: Blockade of B cell activation, IgM and IgG antibody production with Compound (I) treatment

Compound (I) can inhibit B-cell activation, BCR dependent B-cell proliferation, and de novo antibody production. To determine the functional activity of Compound (1), the ability of the compound to inhibit B-cell activation as assessed by anti-IgM-induced CD69 expression in CD20+ B-cells in human whole blood was determined (FIG. 7). The potency of Compound (I) (rilzabrutinib) in the B-cell activation assay was well correlated with BTK target occupancy. The IC₅₀ determinations were 126 ± 32 nM and 233 ± 75 nM for inhibition of CD69 B-cell expression and BTK target occupancy, respectively. As an additional measure of the effects of Compound (I) on B-cell function, the ability of Compound (I) to inhibit B-cell receptor (BCR)-induced human B-cell proliferation was determined. The IC₅₀ of Compound (I) for inhibition of human B-cell proliferation was 5 ± 2.4 nM. Compound (1) did not block antibody-dependent cell-mediated cytotoxicity (ADCC) in combination with anti-CD20 antibodies, indicating potential for combination therapies (Pirunsam, A., P. Kijrattanakul, S. Chamnanchanunt, C. Polprasert, and P. Rojnuckarin. 2018. A randomized multicenter trial comparing low-dose prednisolone versus observation for prevention of recurrences in adult immune thrombocytopenia. Clin Appl Thromb Hemost 24: 867-873.).

Compound (I) significantly inhibited IgM and IgG antibody production when stimulated *in vitro.* Compound (I) inhibited IgG antibody production with an IC₅₀ of 20 ± 20 nM and IgM antibody production with an IC₅₀ of 800 ± 1000 nM when stimulated by a T-cell dependent pathway with anti-CD40 in combination with IL-21 (Fig. 8). Compound (I) also inhibited IgG and IgM antibody production when stimulated by T-independent pathways with the TLR-9 agonist CpG. with an IgG IC₅₀ of 50 ± 90 nM and IgM IC₅₀ of 1 ± 1 nM, as well as with TNP-LPS-stimulated antibody production with IgG IC₅₀ of 300 ± 600 nM and IgM IC₅₀ of 200 ± 600 nM (Table 1).

**Table 1:IC₅₀ Profiles**

| **IC₅₀ (nM)** | **IgG** | **IgM** |
|---|---|---|
| αCD40 + IL-21 | 20 ± 20 | 800 ± 1000 |
| CpG | 50 ± 90 | 1 ± 1 |
| TNP-LNS | 300 ± 600 | 200 ± 600 |

Further general aspects of the invention:
1. A method of treating a disease chosen from membranous nephropathy (MN), IgG4-related diseases (IgG4-RD), and antiphospholipid syndrome (APS) in a mammal in need thereof, comprising administering to said mammal a pharmaceutical composition comprising:
   a compound chosen from (E) isomer, (Z) isomer, and a mixture of (E) and (Z) isomers of 2-[(3R)-3-[4-amino-3-(2-fluoro-4-phenoxy-phenyl)pyrazolo[3,4-d]pyrimidin-1-yl[piperidine-1-carbonyl]-4-methyl-4-[4-(oxetan-3-yl)piperazin-1-yl]pent-2-enenitrile; and/or
   a pharmaceutically acceptable salt of any of the foregoing compounds; and
   a pharmaceutically acceptable carrier or excipient.
2. The method of aspect 1, wherein the disease is acute.
3. The method of aspect 1 or 2, wherein the disease is an IgG4-RD disease flare.
4. The method of any one of aspects 1-3, wherein the pharmaceutical composition is orally administered.
5. The method of any one of aspects 1-4, wherein the pharmaceutical composition is administered daily.
6. The method of any one of aspects 1-5. wherein the pharmaceutical composition is orally administered daily.
7. The method of any one of aspects 1-6, wherein the pharmaceutical composition is administered in place of an immunosuppressive therapy.
8. The method of any one of aspects 1-7, wherein the pharmaceutical composition is administered in place of a corticosteroid therapy.
9. The method of any one of aspects 1-6, wherein the pharmaceutical composition is administered in combination with an immunosuppressive therapy.
10. The method of any one of aspects 1-6, wherein the pharmaceutical composition is administered in combination with a corticosteroid therapy.
11. The method of any of aspects 1-6 or 8-10, wherein the pharmaceutical composition is administered in combination with a noncorticosteroidal immunosuppressive and/or anti-inflammatory agent.
12. The method of any one of aspects 1 or 4-6, wherein the pharmaceutical composition is administered in place of an immunosuppressive maintenance therapy.
13. The method of any one of aspects 1 or 4-6, wherein the pharmaceutical composition is administered in place of a corticosteroid maintenance therapy.
14. The method of any one of aspects 1, 2, or 4-6, wherein the pharmaceutical composition is administered in combination with an immunosuppressive maintenance therapy.
15. The method of any one of aspects 1. 2. or 4-6. wherein the pharmaceutical composition is administered in combination with a corticosteroid maintenance therapy.
16. The method of any one of aspects 13-15, wherein the pharmaceutical composition is administered in combination with a noncorticosteroidal immunosuppressive and/or anti-inflammatory agent.
17. The method of any one of aspects 1- 16, wherein the pharmaceutical composition comprises a compound which is a substantially pure (E) or (Z) isomer of 2-[(3R)-3-|4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile, and/or
   a pharmaceutically acceptable salt of said compound; and
   a pharmaceutically acceptable carrier or excipient.
18. The method of any one of aspects 1-17. wherein at least about 85% w/w of 2-|(3R)-3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile or at least about 85% w/w of a pharmaceutically acceptable salt of 2-[(3R)-3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperarin-1-yl]pent-2-enenitrile is the (E) isomer.
19. The method of any one of aspects 1-18, wherein the mammal is a human.
20. The method of any one of aspectsl-19, wherein the disease is an IgG4-related disease characterized by an IgG4-RD Responder Index (RI) greater than or equal to 2 in at least one organ system.
21. The method of any one of aspects 1-20, wherein the disease is an IgG4-related disease characterized by a serum IgG4 concentration greater than 1.5 times the upper limit of normal.
22. The method of any one of aspects 1-21, wherein the disease is chosen from IgG4-related sialadenitis, IgG4-related ophthalmic disease (IgG4-ROD), IgG4-related pharyngitis, IgG4-related thyroid disease, IgG4-related hypophysitis, IgG4-related pachymeningitis. IgG4-related leptomeningitis, IgG4-related pancreatitis. IgG4-related lung disease, IgG4-related pleuritis, IgG4-related hepatopathy, IgG4-related sclerosing cholangitis, IgG4-related cholecystitis, IgG4-related aortitis, IgG4-related periaortitis, IgG4-related periarteritis, IgG4-related pericarditis, IgG4-related mediastinitis, IgG4-related retroperitoneal fibrosis, IgG4-related mesenteritis, IgG4-related mastitis, IgG4-related kidney disease (IgG4-RKD). IgG4-related prostatitis, IgG4-related perivasal fibrosis, IgG4-related paratesticular pseudotumor, IgG4-related epididymo-orchitis, IgG4-related lymphadenopathy, IgG4-related skin disease. and IgG4-related perineural disease, or flares of any of the preceding IgG4-related diseases.
23. The method of aspect 1, wherein the pharmaceutical composition is administered in combination with an active pharmaceutical ingredient chosen from interferon alpha, interferon gamma, cyclophosphamide, tacrolimus, mycophenolate mofetil, methotrexate, dapsone, sulfasalazine, azathioprine, an anti-CD20 agent, an anti-TNF alpha agent, an anli-IL-4 agent toward ligand or its receptors, an anti-IL-6 agent toward ligand or its receptors, an anti-IL-12 agent to ligand or its receptors, an anti-IL-17 agent to ligand or its receptors, an anti-IL-1 agent to ligand or its receptors, an anti-IL-2 agent to ligand or its receptors, an anti-IL-23 agent to ligand or its receptors, an anti-IL-12/23 agent to ligand or receptors, an anti-CD2 agent, an anti-CD3 agent, an anti-CD80/86 agent, an anti-sphingosine-1-phosphate receptor agent, an anti-C5 agent, an anti-mTOR agent, an anti-calcineurin agent, an anti-BAFF/BlyS agent, leflunomide, and teriflunomide.
24. The method of aspect 1, wherein the pharmaceutical composition is administered in combination with rituximab, dupilumab, ofatumumab, obinutuzumab, or veltuzumab, or a biosimilar version of any of the foregoing.
25. The method of any one of aspects 1-6, 9-11, or 14-24, wherein the mammal is treatment naïve with respect to a immunosuppressive therapy.
26. The method of any one of aspects 1-6, 9-11, or 14-24, wherein the mammal is treatment naive with respect to any immunosuppressive therapy.
27. The method of any one of aspects 1-24, wherein the mammal has been previously treated with an immunosuppressive therapy.

## Claims

1. A compound chosen from (E) isomer, (Z) isomer, and a mixture of (E) and (Z) isomers of 2-[(3R)-3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile, or a pharmaceutically acceptable salt of any of the foregoing compounds, for use in a method of treating a disease chosen from membranous nephropathy (MN) and IgG4-related disease (IgG4-RD) in a mammal in need thereof.

2. The compound for use according to claim 1, wherein the disease is acute.

3. The compound for use according to claim 1 or 2, wherein the disease is an IgG4-RD disease flare.

4. The compound for use according to any one of claims 1 to 3, wherein the compound is orally administered.

5. The compound for use according to any one of claims 1 to 4, wherein the compound is administered in combination with an immunosuppressive therapy or a corticosteroid therapy.

6. The compound for use according to any one of claims 1 to 5, wherein the compound is administered in combination with a noncorticosteroidal immunosuppressive and/or anti-inflammatory agent.

7. The compound for use according to any one of claims 1 to 6, wherein the compound is administered in combination with an immunosuppressive maintenance therapy or a corticosteroid maintenance therapy.

8. The compound for use according to any one of claims 1 to 7, comprising an (E) or (Z) isomer of 2-[(3R)-3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile, the compound having at least 70% by weight of the (E) isomeric form or having at least 70% by weight of the (Z) isomeric form, or a pharmaceutically acceptable salt of said compound.

9. The compound for use according to any one of claims 1 to 8, wherein at least 85% ± 10% w/w of 2-[(3R)-3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile or at least 85% ± 10% w/w of a pharmaceutically acceptable salt of 2-[(3R)-3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile is the (E) isomer.

10. The compound for use according to any one of claims 1 to 9, wherein the mammal is a human.

11. The compound for use according to any one of claims 1 to 10, wherein the disease is chosen from IgG4-related sialadenitis, IgG4-related ophthalmic disease (IgG4-ROD), IgG4-related pharyngitis, IgG4-related thyroid disease, IgG4-related hypophysitis, IgG4-related pachymeningitis, IgG4-related leptomeningitis, IgG4-related pancreatitis, IgG4-related lung disease, IgG4-related pleuritis, IgG4-related hepatopathy, IgG4-related sclerosing cholangitis, IgG4-related cholecystitis, IgG4-related aortitis, IgG4-related periaortitis, IgG4-related periarteritis, IgG4-related pericarditis, IgG4-related mediastinitis, IgG4-related retroperitoneal fibrosis, IgG4-related mesenteritis, IgG4-related mastitis, IgG4-related kidney disease (IgG4-RKD), IgG4-related prostatitis, IgG4-related perivasal fibrosis, IgG4-related paratesticular pseudotumor, IgG4-related epididymo-orchitis, IgG4-related lymphadenopathy, IgG4-related skin disease, and IgG4-related perineural disease, and flares of any of the preceding IgG4-related diseases.

12. The compound for use according to claim 1, wherein the compound is administered in combination with an active pharmaceutical ingredient chosen from interferon alpha, interferon gamma, cyclophosphamide, tacrolimus, mycophenolate mofetil, methotrexate, dapsone, sulfasalazine, azathioprine, an anti-CD20 agent, an anti-TNF alpha agent, an anti-IL-4 agent toward ligand or its receptors, an anti-IL-6 agent toward ligand or its receptors, an anti-IL-12 agent to ligand or its receptors, an anti-IL-17 agent to ligand or its receptors, an anti-IL-1 agent to ligand or its receptors, an anti-IL-2 agent to ligand or its receptors, an anti-IL-23 agent to ligand or its receptors, an anti-IL-12/23 agent to ligand or receptors, an anti-CD2 agent, an anti-CD3 agent, an anti-CD80/86 agent, an anti-sphingosine-1-phosphate receptor agent, an anti-C5 agent, an anti-mTOR agent, an anti-calcineurin agent, an anti-BAFF/BlyS agent, leflunomide, and teriflunomide.

13. The compound for use according to claim 1, wherein the compound is administered in combination with rituximab, dupilumab, ofatumumab, obinutuzumab, or veltuzumab, or a biosimilar version of any of the foregoing.

14. The compound for use according to any one of claims 1 to 13, wherein the mammal is treatment naive with respect to an immunosuppressive therapy.

15. The compound for use according to any one of claims 1 to 13, wherein the mammal has been previously treated with an immunosuppressive therapy.
